Europäisches Patentamt

European Patent Office

Office européen des brevets

(11) Veröffentlichungsnummer : **0 196 409**
**B1**

(12) **EUROPÄISCHE PATENTSCHRIFT**

(45) Veröffentlichungstag der Patentschrift :
**11.01.89**

(21) Anmeldenummer : **86100695.5**

(22) Anmeldetag : **20.01.86**

(51) Int. Cl.⁴ : **A 61 F   2/38**, A 61 F   2/34

(54) **Kappen- oder schalenartige Gelenkendoprothese.**

(30) Priorität : **19.03.85 CH 1220/85**

(43) Veröffentlichungstag der Anmeldung :
**08.10.86 Patentblatt 86/41**

(45) Bekanntmachung des Hinweises auf die Patenterteilung : **11.01.89 Patentblatt 89/02**

(84) Benannte Vertragsstaaten :
**AT DE FR GB IT**

(56) Entgegenhaltungen :
**EP--A-- 0 013 864**
**CH--A--   463 016**
**CH--A--   514 078**
**DE--A-- 2 247 560**
**DE--A-- 3 310 944**
**GB--A-- 1 080 751**
**GB--A-- 1 159 049**
**US--A-- 3 477 335**
**US--A-- 3 842 824**

(73) Patentinhaber : **GEBRÜDER SULZER AKTIENGESELL-SCHAFT**
**Zürcherstrasse 9**
**CH-8401 Winterthur (CH)**

(72) Erfinder : **Frey, Otto**
**Wallrütistrasse 56**
**CH-8400 Winterthur (CH)**.
Erfinder : **Koch, Rudolf**
**Oberdorfstrasse 229**
**CH-8267 Berlingen (CH)**

(74) Vertreter : **Dipl.-Ing. H. Marsch Dipl.-Ing. K. Sparing Dipl.-Phys.Dr. W.H. Röhl Patentanwälte**
**Rethelstrasse 123**
**D-4000 Düsseldorf 1 (DE)**

EP 0 196 409 B1

## Beschreibung

Die Erfindung betrifft eine kappen- oder schalenartige Gelenkendoprothese, bei der mindestens zwei Verankerungselemente für eine Fixierung im Knochen durch die Gleitfläche des Gelenkes hindurch verlaufen und mit ihren Achsen schiefwinklig zueinander und/oder zur Einsetzrichtung der Prothese angeordnet sind.

Derartige Prothesen sind z. B. eine Schlitzprothese für die Oberschenkel-kondylen eines Kniegelenks, bei der die beiden « Kufen » einzeln in dem Knochen verankert werden. Wegen der schiefen Stellung der Verankerungsrichtung zueinander müssen die Verankerungselemente durch die Gleitfläche des Gelenkes hindurch im Knochen fixiert werden. In bekannter Weise werden derartige Prothesen daher in den Knochen eingeschraubt. Um die notwendige Haftung zu erreichen, sind für die Knochenschrauben relativ grosse Gewindesteigungen und als Folge davon relativ grosse Kopfdurchmesser erforderlich. Diese verursachen relativ grosse « Störungen » in den Gleitflächen der Prothese für das Gewinde und den Schraubenkopf. Weiterhin sind derartige Knochenschrauben für Biegebelastungen ungeeignet.

Aufgabe der Erfindung ist es somit, Prothesen der genannten Art zu schaffen, bei denen die « Eingriffe » in die Gleitflächen so gering wie möglich sind, und die Verankerungselemente auch auf Biegung beansprucht werden können.

Mit der vorliegenden Erfindung wird diese Aufgabe dadurch gelöst, dass die Verankerungselemente aus einer aufspreizbaren Hülse bestehen, die an einem Ende einen hohlen Kopf trägt, dessen Aussendurchmesser grösser ist als derjenige ihres in an sich bekannter Weise mit Längsschlitzen versehenen Körpers, wobei der Kopf auf seinem äusseren Umfang ein Aussengewinde trägt, sowie Mittel für den Eingriff eines Schraubwerkzeuges aufweist, und dass ferner in der Gleitfläche entsprechende Gewindebohrungen für den Hülsenkopf vorgesehen sind.

Aufspreizbare Hülsen als Verankerungselemente für die Lagerpfanne einer eine Kunststoff-Gleitpfanne aufweisenden prothetischen Pfanne für ein Körpergelenk sind bereits aus der DE-A-3 310 944 bekannt. Die Hülsentragen an einem Ende einen hohlen Kopf, dessen Außendurchmesser größer als derjenige ihres mit einem Schlitz versehenen Körpers ist. In die Hülsen sind Spreizschrauben einsetzbar. Die Kunststoff-Gleitpfanne und formschlüssig in die Lagerpfanne eingeschnappt.

Bei der erfindungsgemäßen Lösung können die durch Aufspreizen verankerten Hülsen, die aussen mit Zirkularrippen versehen sein können, relativ zu Knochenschrauben mit geringen Durchmessern versehen werden ; sie können in vorgefertigte Bohrungen eingesetzt werden, ohne dass, wie bei einer Knochenschraube, sich ein Gewinde in das spongiose Gewebe schneidet. Weiterhin ist vor allem der von dem mit der Prothese verschraubten Hülsenkopf beanspruchte Gleitflächenanteil ein Bruchteil von demjenigen für den Kopf einer Knochenschraube. Im Gegensatz zu Knochenschrauben sind, wie gefordert, aufspreizbare Hülsen auch durch Biegungen belastbar, ohne dass die Verankerung beeinträchtigt wird.

Ein Aufspreizen der Hülse kann vorteilhafterweise mittels eines zum Kopf hin in sie einschiebbaren konischen Spreizkörpers erfolgen, der über eine Sollbruchstelle mit einem die Hülse und ihren Kopf durchsetzenden Zugstab verbunden ist ; zusätzlich kann der Spreizkörper dabei am Ende seines Konus einen Einrastansatz aufweisen.

Eine weitere Verkleinerung der « Störung » in der Gleitfläche lässt sich erreichen, wenn eine der Gewindebohrungen auf der dem Knochen zugewandten Rückseite der Prothesenschale oder -kappe angeordnet ist, da bei der von der Rückseite eingeschraubten Hülse die Oeffnung in der Gleitfläche etwa auf den Durchmesser des Zugstabes verringert werden kann. Schliesslich ist es für eine Zentrierung der Prothese relativ zu den vorgebohrten Löchern für die Spreizhülse zweckmässig, wenn die Rückseite der Prothese um die Gewindebohrungen herum mit Zentrierkonen oder Zentrierkugelflächen versehen ist.

Die Erfindung wird im folgenden anhand eines Ausführungsbeispiels im Zusammenhang mit der Zeichnung näher erläutert.

Fig. 1 ist ein Sagittalschnitt durch eine Femurkondyle, in der eine Kufe einer Schlittenprothese verankert ist ;

Fig. 2 stellt vergrössert einen Längsschnitt der Verankerungshülse dar, während ;

Fig. 3 einen Zugstab, ebenfalls vergrössert, wiedergibt.

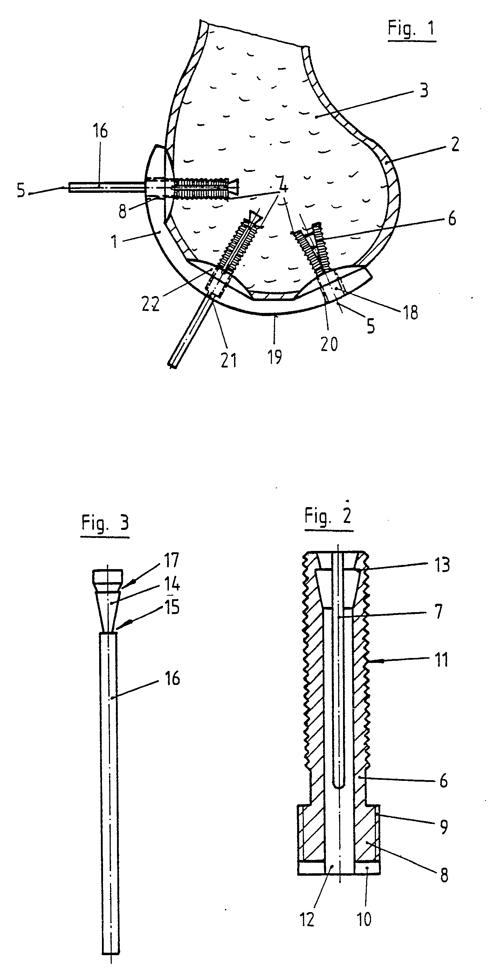

In Fig. 1 ist eine plattenartige Prothese 1 gezeigt, die als Ersatz für die natürliche Kondylengleitfläche im Femur 2 befestigt ist. Die Verankerung der Prothese 1 erfolgt in der Spongiosa 3 über drei Verankerungselement 4, die mit ihren Achsen 5 schiefwinklig zueinander angeordnet sind.

Die Verankerungselemente 4 bestehen aus mit Längsschlitzen 7 versehenen aufspreizbaren Metallhülsen 6, beispielsweise aus Titan oder einer Titanlegierung ; an einem Ende besitzt die Hülse 6 einen Kopf 8, der ein Aussengewinde 9 trägt und in seiner Stirnfläche einen Schlitz 10 für den Eingriff eines nicht gezeigten Schraubwerkzeugs hat. Der Aussenmantel des Hülsenkörpers ist mit relativ scharfkantigen zirkularen Rippen 11 versehen, die sich beim Aufspreizen der Hülse 6 in die Spongiosa 3 einpressen.

In Richtung ihrer Längsachse ist die Hülse 6 durchbohrt, wobei die Bohrung 12 am « Fussende » der Hülse 6 zwei konische Erweiterungen aufweist, durch die eine Einrastvertiefung 13 für einen Spreizkörper 14 (Fig. 3) geschaffen wird.

Der Spreizkörper 14 sitzt über einer Sollbruchstelle 15 an einem Zugstab 16, mit dessen Hilfe er zum Aufspreizen der Hülse 6 nach Einsetzen des Implantats 1 und Montage der jeweiligen Hülse 6 gegen den Kopf 8 der Hülse 6 gezogen wird, bis er in die Vertiefung 13 mit einem entsprechenden Absatz 17 einrastet. Durch Abbrechen oder Abreissen an der Sollbruchstelle 15 wird der Zugstab 16 entfernt. Die Darstellung der Fig. 1 zeigt rechts eine bereits aufgespreizte Hülse 6 mit eingezogenem Spreizkörper 14, während die beiden anderen Verankerungen die Hülse 6 mit einem sie durchsetzenden Zugstab 16 — also während der Operation — verdeutlichen.

Wie Fig. 1 zeigt, sind die Köpfe 8 der Hülsen 6 in Gewindebohrungen 18 der Prothese 1 eingeschraubt. Bei der gezeigten Ausführungsform durchsetzen dabei die beiden äusseren Gewindebohrungen 18 das Implantat 1 von seiner Gleitfläche 19 her, während die mittlere Bohrung 18 mit dem den Gewindekopf aufnehmenden Gewinde 20 von der Rückseite her in das Implantat 1 hineinführt. Bei diesem mittleren Verankerungselement 4 wird die Gleitfläche 19 lediglich von einer relativ kleinen Bohrung 21 für den Durchtritt des Zugstabes 16 durchstossen.

Die Bohrungen 18 sind auf der Rückseite des Implantats 1 mit Zentrierkugelflächen 22 versehen, durch die eine genaue Zentrierung der Bohrungen 18 auf in die Spongiosa 3 eingebrachte Bohrungen für die Hülsen 6 erleichtert wird ; die Zentrierung kann dabei auch statt über Zentrierkugelflächen mit Hilfe von Zentrierkonen erfolgen. Weiterhin kann die Rückseite der Prothese 1 mit das Anwachsen von Gewebe fördernden Strukturen versehen sein, was nicht ausdrücklich dargestellt ist.

**Patentansprüche**

1. Kappen- oder schalenartige Gelenkendoprothese (1), bei der mindestens zwei Verankerungselemente (4) für eine Fixierung im Knochen (2, 3) durch die Gleitfläche (19) des Gelenkes hindurch verlaufen und mit ihren Achsen schiefwinklig zueinander und/oder zur Einsetzrichtung der Prothese angeordnet sind, dadurch gekennzeichnet, dass die Verankerungselemente (4) aus einer aufspreizbaren Hülse (6) bestehen, die an einem Ende einen hohlen Kopf (8) trägt, dessen Aussendurchmesser grösser ist als derjenige ihres in an sich bekannter Weise mit Längsschlitzen (7) versehenen Körpers, wobei der Kopf auf seinem äusseren Umfang ein Aussengewinde (9) trägt, sowie Mittel (10) für den Eingriff eines Schraubwerkzeugs aufweist, und dass ferner in der Gleitfläche (19) entsprechende Gewindebohrungen (18) für den Hülsenkopf (8) vorgesehen sind.

2. Gelenkendoprothese nach Anspruch 1, dadurch gekennzeichnet, dass die Spreizung der Hülse (6) mittels eines zum Kopf (8) hin in sie einziehbaren konischen Spreizkörpers (14) erfolgt, der über eine Sollbruchstelle (15) mit einem die Hülse (6) und ihren Kopf (8) durchsetzenden Zugstab (16) verbunden ist.

3. Gelenkendoprothese nach Anspruch 2, dadurch gekennzeichnet, dass der Spreizkörper (14) am Ende seines Konus einen Einrastansatz (17) besitzt.

4. Gelenkendoprothese nach Anspruch 2, dadurch gekennzeichnet, dass eine der Gewindebohrungen (18) auf der dem Knochen (2) zugewandten Rückseite der Prothesenschale (1) oder -kappe angeordnet ist.

5. Gelenkendoprothese nach Anspruch 1, dadurch gekennzeichnet, dass die Rückseite der Prothese (1) um die Gewindebohrungen (18) herum mit Zentrierkonen oder Zentrierkugelflächen (22) versehen ist.

**Claims**

1. A cap-like or shell-like joint replacement (1) in which at least two fixing elements (4) for fixing in the bone (2, 3) extend through the rubbing surface (19) of the joint and have their axes disposed in skewed relationship to one another and/or to the direction of insertion of the replacement, characterised in that the fixing elements are in the form of an expanding tube (6) having at one end a hollow head (8) whose outer diameter is greater than that of the tube body, the same being formed with longitudinal slots (7) in known manner, the head having an external screwthread (9) on its outer periphery and means (10) engageable by a screwdriver device ; and the rubbing surface (19) is formed with companion tapped bores (18)· for the tube head (8).

2. A replacement according to claim 1, characterised in that the tube (6) is expanded by means of a conical expander (14) which is adapted to be drawn into the tube (6) towards the head (8) and which is connected by way of a frangible zone (15) to a draw rod (16), the same extending through the tube (6) and head (8).

3. A replacement according to claim 2, characterised in that the expanding member (14) has a catch projection (17) at the end of its cone.

4. A replacement according to claim 2, characterised in that one of the tapped bores (18) is disposed on the back of the replacement (1), the back being near the bone (2).

5. A replacement according to claim 1, characterised in that the back of the replacement (1) has centering cones or centering spherical surfaces (22) disposed around the tapped bores (18).

**Revendications**

1. Endoprothèse d'articulation (1) en forme de capuchon ou de coque, dans laquelle deux éléments d'ancrage (4) au moins assurant une fixation dans l'os (2, 3) traversent la surface de glissement (19) de l'articulation, et dont les axes sont obliques l'un par rapport à l'autre et/ou par rapport à la direction d'implantation de la prothèse, caractérisée en ce que les éléments d'ancrage (4) sont constitués d'une douille (6) à expansion qui porte à une extrémité une tête (8) creuse dont le diamètre extérieur est supérieur à celui de son corps pourvu d'une manière connue de fentes longitudinales (7), la tête portant sur son pourtour extérieur un filetage (9) ainsi que des moyens (10) de prise pour un outil de vissage, et en ce que des trous taraudés (18) correspondants sont pratiqués dans la surface de glissement (19) pour la tête (8) de la douille.

2. Endoprothèse d'articulation selon la revendication 1, caractérisée en ce que l'expansion de la douille (6) s'effectue au moyen d'un corps d'écartement (14) conique à introduire dans celle-ci vers la tête (8), ce corps d'écartement étant relié par un point de rupture (15) à un tirant (16) traversant la douille (6) et sa tête (8).

3. Endoprothèse d'articulation selon la revendication 2, caractérisée en ce que le corps d'écartement (14) présente un épaulement d'encliquetage (17) à l'extrémité de son cône.

4. Endoprothèse d'articulation selon la revendication 2, caractérisée en ce que l'un des trous taraudés (18) est disposé sur la face arrière de la coque (1) ou du capuchon de la prothèse, tournée vers l'os (2).

5. Endoprothèse d'articulation selon la revendication 1, caractérisée en ce que la face arrière de la prothèse (1) est pourvue de cônes de centrage ou de surfaces sphériques de centrage (22) autour des trous taraudés (18).

Fig. 1

Fig. 3

Fig. 2